(19)

(11) **EP 2 335 659 B1**

(12) **EUROPEAN PATENT SPECIFICATION**

(45) Date of publication and mention of the grant of the patent:
**06.06.2012 Bulletin 2012/23**

(21) Application number: **09814633.5**

(22) Date of filing: **17.09.2009**

(51) Int Cl.:
***A61F 5/455*** *(2006.01)* ***A61F 5/44*** *(2006.01)*
***A61F 5/451*** *(2006.01)* ***A61F 13/15*** *(2006.01)*
***A61F 13/42*** *(2006.01)*

(86) International application number:
**PCT/JP2009/066265**

(87) International publication number:
**WO 2010/032789 (25.03.2010 Gazette 2010/12)**

(54) **URINE RECEIVER AND WEARING ARTICLE**

URINFÄNGER UND KLEIDUNGSSTÜCK DAMIT

COLLECTEUR D'URINE ET ARTICLE A PORTER

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **19.09.2008 JP 2008241776**
**19.09.2008 JP 2008241778**
**19.09.2008 JP 2008241779**

(43) Date of publication of application:
**22.06.2011 Bulletin 2011/25**

(60) Divisional application:
**11167990.8 / 2 374 435**
**11168588.9 / 2 380 532**

(73) Proprietor: **Uni-charm Corporation**
**Ehime 799-0111 (JP)**

(72) Inventors:
- **WADA, Ichiro**
  **Kanonji-shi**
  **Kagawa 769-1602 (JP)**
- **SUZUKI, Miou**
  **Kanonji-shi**
  **Kagawa 769-1602 (JP)**
- **YAMAKI, Rumi**
  **Kanonji-shi**
  **Kagawa 769-1602 (JP)**
- **MURAKAMI, Hiroko**
  **Kanonji-shi**
  **Kagawa 769-1602 (JP)**

(74) Representative: **Knights, Rupert**
**Saunders & Dolleymore LLP**
**9 Rickmansworth Road**
**Watford WD18 0JU (GB)**

(56) References cited:
**EP-A1- 1 616 542**
**WO-A1-01/45621**
**JP-A- 61 000 342**
**JP-A- 2003 093 438**
**JP-A- 2006 116 348**
**JP-A- 2007 044 493**
**JP-A- 2009 189 499**
**US-A1- 2007 038 194**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 335 659 B1

## Description

### TECHNICAL FIELD

The present invention relates to urine receivers adapted to be put on a person such as a bedridden aged one for whom it is difficult to control timing of urination and/or to make disposal of urine after discharged so that automatic urine disposal may be achieved. Document US 2007/038194A1 is regarded as the closest prior art.

### RELATED ART

Conventionally, automatic urine disposal systems for a physically disabled person such as a bedridden aged one are known. For example, the urine receiver disclosed in JP 2006-26108 A includes a suction unit to collect the urine discharged by the wearer and a urine guide tube extending from the suction unit to a vacuum tank. The suction unit has an opening covered with a liquid-pervious sheet and this liquid-pervious sheet is provided on its inner surface with a pair of electrodes spaced from and extending in parallel to each other. With the urine receiver put on the wearer's body, when the paired electrodes are electrically connected with each other by urine, electric signals are generated and, in response to this electric signal, a vacuum pump is actuated to cause urine evacuation from the suction unit toward the urine guide pipe to be initiated.

### DISCLOSURE OF THE INVENTION

### PROBLEM TO BE SOLVED BY THE INVENTION

In the urine receiver disclosed in JP 2006-26108 A, a pair of electrodes is covered with the liquid-pervious sheet so that, upon occurrence of urination by the wearer of the urine receiver, urine may spread over this liquid-pervious sheet and/or the other liquid-pervious sheets and the paired electrodes may be electrically connected to each other via the liquid-pervious sheet(s) wetted with urine. However, it is not necessarily assured depending on the wearer's posture that the discharged urine smoothly spreads over the liquid-pervious sheet(s) and, in consequence, there is a possibility that the vacuum pump could not be actuated even when urination occurs.

It is an object of the present invention to improve the known urine receiver so that a pair of electrodes may be electrically connected with each other via urine rapidly upon occurrence of urination.

### MEASURE TO SOLVE THE PROBLEM

According to the present invention, there is provided an urine receiver comprising a leakage-barrier receptacle having a bottom and a peripheral wall and a top opening, a urine evacuation duct extending through the peripheral wall adapted to be connected with urine suction means provided separately of the receptacle, a plurality of liquid-pervious sheets stacked one on another to cover the top opening and a pair of electrodes spaced from each other and extending in one direction in parallel to each other wherein, in response to electrical connection of the pair of electrodes via urine contained in one or more of the plurality of liquid-pervious sheets, the urine suction means is actuated to suck urine discharged by the wearer into the receptacle through the top opening and to evacuate the urine sucked into the receptacle from the receptacle through the urine evacuation duct. Any of the plurality of liquid-pervious sheets lying outside the pair of electrodes in a depth direction of the receptacle is formed of a nonwoven fabric containing thermoplastic synthetic fibers and has an outer surface facing outward in the depth direction and an inner surface facing inward in the depth direction.

The present invention is characterized in that the outer surface is formed with undulation comprising crests and troughs alternating in the one direction and each of the crests and troughs extends across the pair of electrodes.

According to one embodiment of the present invention, the sheet formed on the outer surface thereof with the undulation has a flat inner surface and is put in close contact with the liquid-pervious sheet facing the flat inner surface.

According to another embodiment of the present invention, the sheet formed on the outer surface thereof with the undulation has the inner surface extending in parallel to the outer surface and is put in close contact with the liquid-pervious sheet facing the inner surface along respective bottoms of the troughs.

According to still another embodiment of the present invention, respective tops of the crests are hydrophobic and respective bottoms of the troughs are hydrophilic.

According to yet another embodiment of the present invention, the nonwoven fabric contains in the respective troughs thermoplastic synthetic fibers extending in the direction orthogonal to the one direction.

## EFFECT OF THE INVENTION

In the urine receiver according to the present invention, any one of the liquid-pervious sheets covering a pair of electrodes is formed of the nonwoven fabric of which the outer surface has the undulation comprising the crests and the troughs alternating in the direction in which the electrodes extend. These crests and troughs respectively extend in the direction intersecting with the direction in which the electrodes extend across the electrodes. Urine discharged by the wearer of the urine receiver flows into the troughs and flows along the troughs. In consequence, the electrodes can be quickly bridged by urine and electrically turned on. As a result, the urine suction means connected to the urine receiver also can be quickly actuated and suction of urine into the receptacle as well as evacuation of urine from the receptacle can be promoted.

## BRIEF DESCRIPTION OF THE DRAWINGS

Fig. 1 is a schematic diagram illustrating a construction of an automatic urine disposal system including a urine receiver.
Fig. 2 is a diagram showing the urine receiver as put on a wearer.
Fig. 3 is a plan view of the urine receiver.
Fig. 4 is a sectional view taken along line IV-IV in Fig. 3.
Fig. 5 is a sectional view taken along line V-V in Fig. 3.
Fig. 6 is a plan view of a urine sensor.
Fig. 7 is a sectional view taken along line VII-VII in Fig. 6.
Fig. 8 is a plan view of the urine sensor with insulating coating peeled off.
Fig. 9 is a perspective view of a strip of sheet.
Fig. 10 is a sectional view taken along line X-X in Fig. 9.
Fig. 11 is a view similar to Fig. 10, exemplarily showing a skin-contact sheet.

## IDENTIFICATION OF REFERENCE NUMERALS USED IN THE DRAWINGS

| | |
|---|---|
| 100a | urine suction means |
| 102 | urine receiver |
| 112 | urine receptacle (container) |
| 112a | opening |
| 112b | bottom |
| 112c | peripheral wall |
| 114 | opening |
| 143 | electrode |
| 143 | electrode |
| 134 | liquid-pervious sheet (skin-contact sheet) |
| 126 | liquid-pervious sheet (dispersion sheet) |
| 400 | strip of sheet |
| 400a | outer surface (upper surface) |
| 400b | inner surface (lower surface) |
| 401 | crests |
| 402 | troughs |
| 407 | thermoplastic synthetic fibers |
| P | longitudinal direction |
| Q | transverse direction |
| R | depth (thickness) direction |

## DETAILED DESCRIPTION OF THE PREFERRED EMBODIMENTS

Details of a urine receiver according to the present invention will be more fully understood from the description given hereunder with reference to the accompanying drawings.

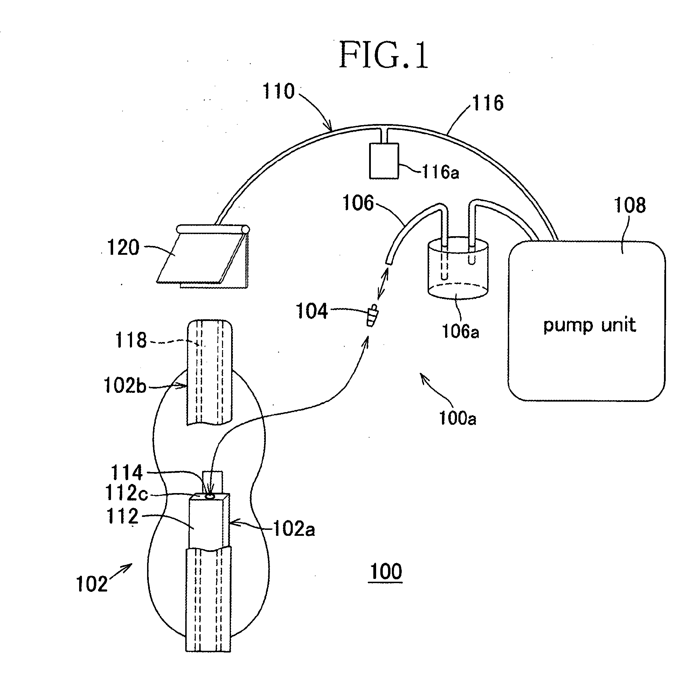

Fig. 1 is a schematic diagram illustrating an automatic urine disposal system 100 comprising a urine receiver 102 according to the present invention and vacuum-utilizing urine suction means 100a. The urine receiver 102 has a side facing the wearer's skin and a side facing the wearer's garment. In Fig. 1, various elements on the side facing the wearer's garment are illustrated as partially cutaway.

The automatic urine disposal system 100 can automatically collect urine discharged by the wearer into the urine receiver 102 upon urination. The urine receiver 102 comprises a urine collector unit 102a put in contact with the wearer's skin in the vicinity of the wearer's urethral orifice and adapted to collect discharged urine and a urine sensor unit 102b adapted to detect urination. The vacuum-utilizing urine suction means 100a includes a joint element 104 by which the suction means 100a is connected to the urine receiver 102, a urine guide tube 106, a urine reservoir 106a, a pump unit 108 and an electric wiring 116.

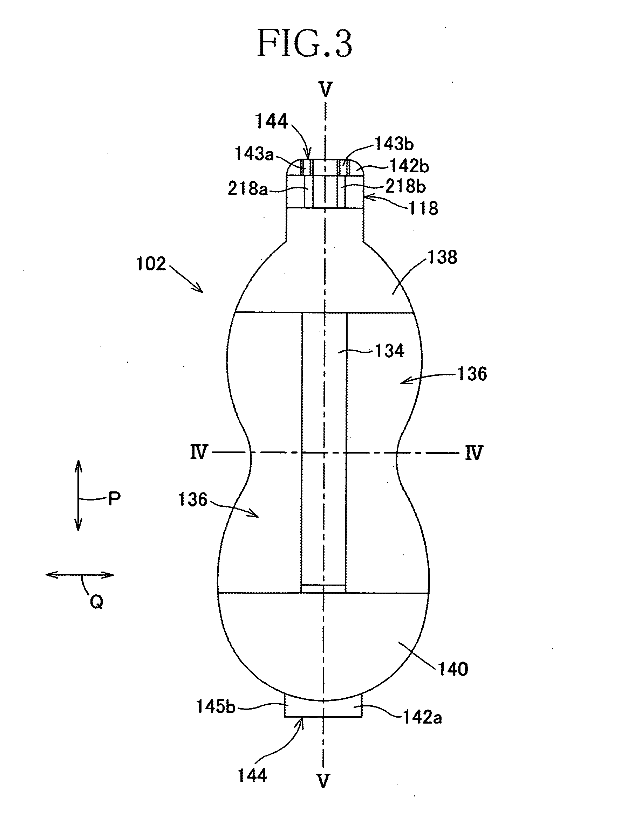

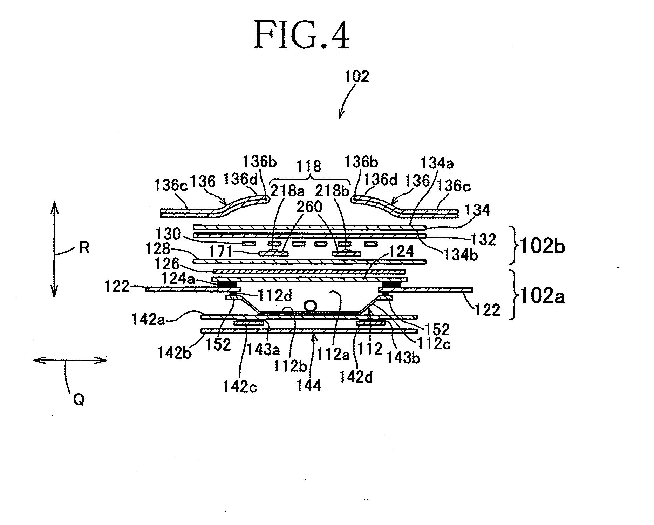

The pump unit 108 includes a suction pump (not shown) adapted to be actuated in response to signals transmitted via the wiring 116 from the urine sensor unit 102b. In the urine receiver 102, the urine guide tube 106 is connected via the joint element 104 to a urine evacuation port 114 formed through a peripheral wall 112c of a receptacle 112 in the urine collector unit 102a. A distal end of the wiring 116 extending from the pump unit 108 is provided with a clip 120 by which a pair of electrodes 218a, 218b (See Figs. 3 and 4) of a urine sensor 118 included by the urine sensor unit 102b are electrically connected to the wiring 116. In such automatic urine disposal system 100, the urine sensor 118 detects occurrence of urination and generates signals on the basis thereof. In response to the signals transmitted from the urine sensor 118, the suction pump included by the pump unit 108 is actuated to suck air within the urine reservoir 106a and thereby to suck urine into the urine receptacle 112 so that the urine sucked in this manner may be further sucked and collected through the joint element 104 and the urine guide tube 106 into the urine reservoir 106a.

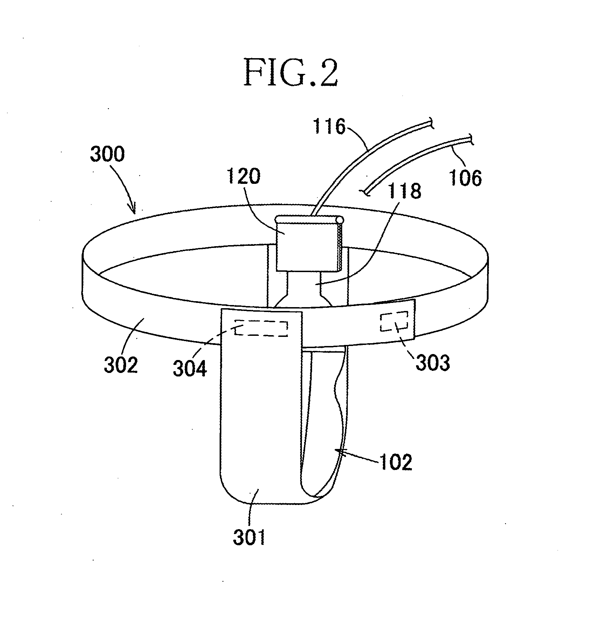

Fig. 2 exemplarily illustrates how to wear the urine receiver 102. With the urine receiver 102 put on the wearer's body, the clip 120 lies on the wearer's ventral side. A T-belt 300 is used as means to wear the urine receiver 102 on the wearer's body, the urine receiver 102 is fixed to the inner surface of a crotch belt segment 301 as a component of the T-belt 300 with pressure-sensitive adhesives or mechanical fasteners well known by the trade mark "Velcro" (registered trademark). Most part of the receptacle 112 in the urine receiver 102 extends in the vertical direction along the front side of the wearer's body so that the inner side of the urine receiver 102 may face the urethral orifice, the vicinity thereof and further extends along the inner surface of the crotch-belt segment 301 toward the anus in a gentle curve. Opposite ends of a waist-belt segment 302 as another component of the T-belt 300 are temporarily connected to each other by suitable connector means 303 such as mechanical fasteners. The crotch-belt segment 301 is stitched at one end together with the waist-belt segment 302 and temporarily connected at the other end to the waist-belt segment 302 with mechanical fasteners 304. It should be noted that the means to wear the urine receiver 102 is not limited to the T-belt 302 as illustrated, but the other appropriate means such as open-type diapers, pants-type diapers, diaper chassises or pants for incontinent patient also may be used for the same purpose.

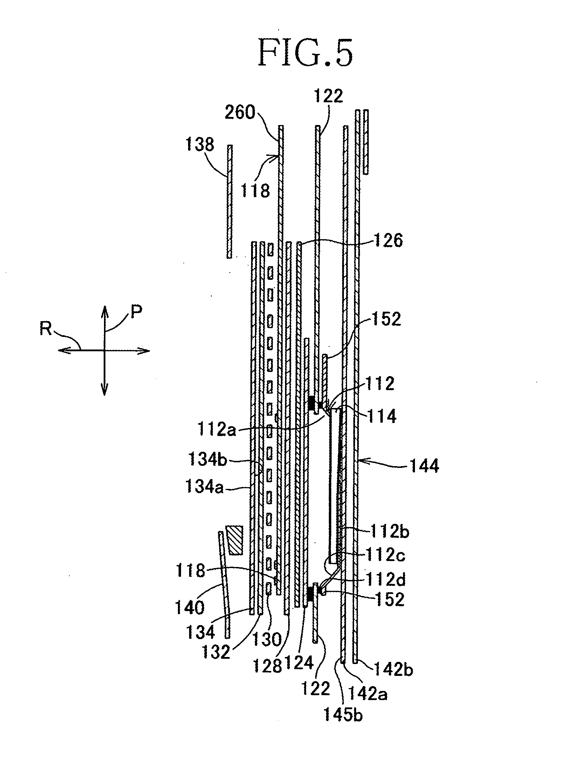

Fig. 3 is a plan view showing the side of the urine receiver 102 facing the wearer's skin (not shown), Fig. 4 is a sectional view taken along line IV-IV in Fig. 3 and Fig. 5 is a sectional view taken along line V-V in Fig. 3. In Figs. 4 and 5, most of respective elements of the urine receiver 102 overlapping one another in the thickness direction R are shown to be spaced one from another for convenience of explanation. The thickness direction R may be reworded by the depth direction of the receptacle 112. As used herein, the term "outward in the depth direction" means the direction from the opening 112a of the receptacle 112 to the outside of the receptacle 112 and as used herein, the term "inward in the depth direction" means the direction from the opening 112a to the inside of the receptacle 112 as viewed in Fig. 4.

The urine receiver 102 has a longitudinal direction P corresponding to the vertical direction of the wearer's body and a transverse direction Q which is orthogonal to the longitudinal direction P. The urine receiver 102 has a width which is relative large in the vicinity of its opposite end sections as viewed in the longitudinal direction P and gradually reduced in its middle section. The urine receiver 102 has a thickness direction R also and includes, above the receptacle 112 as viewed in Fig. 4, a plurality of sheet-like elements, i.e., a liquid-impervious leakage-barrier sheet 122, an air permeable-resistant and liquid-pervious sheet 124, a dispersant sheet 126, a cushion sheet 128, the urine sensor 118, a sheet-like spacer 130, a sheet-like filter 132, a liquid-pervious skin-contact sheet 134 and leakage-barrier 136 stacked in this order from below. The leakage-barrier sheet 122, the air permeable-resistant sheet 124 and the dispersant sheet 126 are integrated with the receptacle 112 to form the urine collector unit 102a. The cushion sheet 128, the urine sensor 118, the spacer 130, the filter 132 and the skin-contact sheet 134 are stacked one on another to form the urine sensor unit 102b.

The receptacle 112 is leak-proof tray-shapedmeans contoured by a bottom 112b, the peripheral wall 112c and an upper opening 112a and made of soft elastic material such as soft polyethylene or silicon rubber. The receptacle 112 has flexibility of a degree such that the receptacle 112 can be inflected in the longitudinal direction P as well as in the transverse direction Q but resist against a deformation due to a negative pressure generated when the suction pump sucks urine. The leakage-barrier sheet 122 is water-tightly bonded to a peripheral flange 152 of the receptacle 112 with adhesive 112d.

The leakage-barrier sheet 122 extends outward beyond the peripheral edge thereof to prevent urine from leaking out of the urine receiver 102. The leakage-barrier sheet 122 may be formed of a thermoplastic synthetic resin film or a composite sheet consisting of such film and a nonwoven fabric. In the case of the urine receiver shown in Fig. 1, a soft polyethylene film having a thickness of 30μm may be used for the leakage-barrier sheet 122. In cases where the urine receiver 102 requires no particular leakage-barrier effect along the periphery of the receptacle 112, the leakage-barrier sheet 22 may be spared from the elements required to exploit the present invention.

The air permeable-resistant sheet 124 is highly liquid-pervious but absolutely or substantially air-impermeable and covers the upper opening 112a of the receptacle 112 as seen in Fig. 4. The periphery of the sheet 124 is bonded to the leakage-barrier sheet 122 from above with hot melt adhesives 124a. A negative pressure can be easily generated within the receptacle 112 covered with the air permeable-resistant sheet 124 sufficiently to suck urine discharged onto the skin-contact sheet 134 rapidly upon actuation of the suction pump of the pump unit 108. The air permeable-resistant sheet 124 may be formed, for example, of an SMS nonwoven fabric consisting of a spun bonded nonwoven fabric having a basis mass of $22g/m^2$, a melt blown nonwoven fabric having a basis mass of $10g/m^2$ and a spun bonded nonwoven fabric having a basis mass of $22g/m^2$ preferably modified by surfactant so as to become hydrophilic. The air-permeability of the air permeable-resistant sheet 124 determined by Air-Permeability Measuring Method A specified by JIS L 1096, Section 6.27.1 is in a range of 0 to $100cc/cm^2/sec$, more preferably in a range of 0 to $50cc/cm^2/sec$ in a wet condition and in a range of 20 to $100cc/cm^2/sec$, more preferably in a range of 20 to $50cc/cm^2/sec$ in a dry condition. The wet condition in which the air-permeability is measured refers to a condition in which the moisture content of the air permeable-resistant sheet 124 calculated according to an equation as indicated below is 100% or higher. The dry condition means the condition of the air permeable-resistant sheet 124 after this sheet 124 has been left for 24 hours or longer in a room at a temperature of 20°C and RH of 50%.

Moisture content (%) = (mass of sheet in wet condition – mass of sheet in a dry condition) / (mass of sheet in a dry condition) … Equation (1)

The dispersant sheet 126 is formed of a liquid-pervious sheet such as a nonwoven fabric containing hydrophilic fibers, for example, rayon fibers and serves to disperse urine over the air permeable-resistant sheet 124 as rapidly as possible so that the air permeable-resistant sheet 124 may be wet over an area as wide as possible. Once the air permeable-resistant sheet 124 has been wetted, it is easy to generate a negative pressure within the receptacle 112 and thereby suck urine into the receptacle 112. Preferably, the dispersant sheet 126 is intermittently bonded to the air permeable-resistant sheet 124 to prevent liquid-pervious properties of these both sheets from being disturbed.

The cushion sheet 128 is formed, for example, of a liquid-pervious sheet such as a thermally bonded nonwoven fabric having a basis mass in a range of 20 to $30g/m^2$ and capable of picking up urine instantaneously at the initial stage of urination to prevent urine staying in the dispersant sheet 126 and the air permeable-resistant sheet 124 from flowing back toward the urine sensor 118. The sheet-like material such as the urine sensor 118, the spacer 130 and the filter 132 may be previously stacked on the cushion sheet 128 to utilize the cushion sheet 128 as a carrier element serving for previously arranging these sheet-like elements at predetermined positions in the urine receiver 102 in the course of producing the urine receiver 102. Preferably, the cushion sheet 128 is intermittently bonded to the dispersant sheet 126 to prevent the liquid-pervious properties of these both sheets from being disturbed.

The urine sensor 118 is obtained, for example, by printing a pair of electrodes each having a desired shape on a synthetic resin film with conductive ink and a detailed construction thereof will be described later. The urine sensor 118 may be located at least in a space defined between the air permeable-resistant sheet 124 and the skin-contact sheet 134 of a plurality of liquid-pervious sheets 124, 126, 128, 130, 132 and 134 and, in the illustrated embodiment, the urine sensor 118 is sandwiched by the cushion sheet 128 and the spacer 130. The urine sensor 118 may be bonded to the cushion sheet 128.

The spacer 130 is formed of a liquid-pervious mesh sheet. In the urine receiver 102, a certain quantity of urine should be left in the skin-contact sheet 134 and keep the skin-contact sheet 134 in a wet condition even after suction of urine. If such skin-contact sheet 134 still in a wet condition comes in direct or indirect contact with the urine sensor 118 under a body mass or the other factors, there is a possibility that the urine sensor might malfunction. The spacer 130 is means serving to keep the urine sensor 118 and the filter 132 to be spaced from each other in the thickness direction R and thereby to prevent such malfunction of the urine sensor 118. The spacer 130 is not urine-absorbent but water-repellent and has air-permeability, liquid-permeability and stiffness higher than those of the air permeable-resistant sheet 124 so that the spacer 130 may keep its thickness unchanged even under the body mass. Such spacer 130 may be formed of a mesh sheet having a thickness in a range of 0.5 to 1mm made of a soft synthetic resin such as ethylene-vinyl acetate and bonded to the cushion sheet 128 preferably in such a manner that the liquid-permeably properties of these both sheets 130, 128 is not disturbed.

The filter 132 serves to avoid a situation in which solid contents of urine might cling to the urine sensor 118 and, in consequence, the urine sensor 118 might be permanently left in a turn-on state. The filter 132 may be formed of a sheet material, more preferably of a nonwoven fabric having air-permeability and liquid-permeability higher than those of the air permeable-resistant sheet 124. The filter 132 may be intermittently bonded to the spacer 130 to prevent the liquid-permeably properties of these both sheets 132, 130 from being disturbed.

The skin-contact sheet 134 has an upper surface 134a and a lower surface 134b wherein the lower surface 134b is kept in tight contact with the upper surface of the filter 132. With the urine receiver 102 put on the wearer's body, the upper surface 134a faces the wearer's urination orifice and the vicinity thereof and, in this state, is kept in contact with the wearer's skin. This skin-contact sheet 134 is formed of a sheet material having flexibility and liquid-permeability such as a thermally bonded nonwoven fabric having a basis mass in a range of 15 to $25 g/m^2$. Like the cushion sheet 128, the skin-contact sheet 134 is capable of picking up urine instantaneously at the initial stage of urination and it is preferred to bond the skin-contact sheet 134 intermittently to the filter 132 so that the liquid-permeable property of these both sheets 134, 132 may be assured. The skin-contact sheet 134 may be hydrophilic or water-repellent depending on conditions.

Paired right and left leakage-barriers 136 seen in Figs. 3 and 4 are capable of preventing urine from flowing on the skin-contact sheet 134 in the transverse direction Q and leaking sideways out of the urine receiver 102. Of the leakage-barriers 136, outer side edges 136c lying outside the urine receiver 102 are bonded to the skin-contact sheet 134 and inner side edges 136d lying in the vicinity of middle section of the urine receiver 102 are not bonded to the skin-contact sheet 134 wherein the inner side edges 136d are provided with elastic elements136b such as rubber yarns (See Fig. 4) are attached thereto under tension in the longitudinal direction P. With the urine receiver 102 put on the wearer's body, the elastic elements 136b are curved in the longitudinal direction P as seen in Fig. 1 and thereupon and the inner side edges 136d of the leakage-barriers 135 are spaced upward from the skin-contact sheet 134. The leakage-barriers 136 are formed of a soft thermoplastic synthetic resin film or a composite sheet consisting of this film and a nonwoven fabric and are preferably liquid-impervious. As viewed in the plan view of the urine receiver 102, the leakage-barriers 136 (See Fig. 3) have upper and lower ends covered with first and second end sheets 138, 140, respectively.

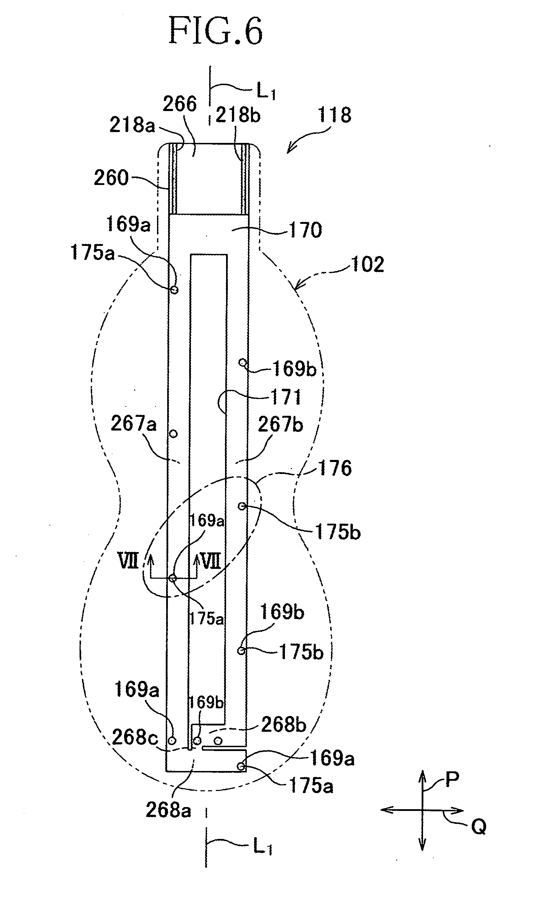

With the urine receiver 102 put on the wearer's body, if defecation occurs and the skin-contact sheet 134 is covered with feces, it will be difficult or sometimes impossible for the urine receiver 102 to detect urination and to start the suction of urine. To solve this problem, the urine receiver 102 is provided with a feces sensor 144 as will be apparent from Figs. 3, 4 and 5. In the feces sensor 144, thermoplastic synthetic resin film sheets 142c, 142d are formed on respective upper surfaces with electrodes 142c, 142d and covered with cover sheets 142a, 142b, respectively. The cover sheet 142a is liquid-pervious and allows moisture in feces to pass therethrough toward the electrodes 143a, 143b. In this feces sensor 144, the electrodes 143a, 143b extend in the longitudinal direction P in parallel to the urine detecting electrodes 218a, 218b and have lower ends 145b as viewed in Figs. 3 and 6 extend downward beyond the second end sheet 140. When the lower ends 145b are soiled with feces, moisture in feces permeates the cover sheet 142a, causing the electrodes 143a, 143b to be electrically connected to each other. Thereupon electric current is supplied from a power source 116a (See Fig. 1) included by the wiring 116 and an alarm device (not shown) in the pump unit 108 receives signals. In response to the signals, the alarm device prompts the care personnel to deal with feces and to exchange the urine receiver 102 with the fresh one.

Fig. 6 is a plan view of the urine sensor 118 used in the embodiment illustrated in Figs. 3, 4 and 5 wherein the contour of the urine receiver 102 is indicated by imaginary line. The urine sensor 118 comprises a film sheet 260 formed of a synthetic resin film, a pair of the urine detecting electrodes 218a, 218b formed on one surface of the film sheet 260 and the insulating coating 170 covering the most part of these electrodes 218a, 218b. The film sheet 260 has a rectangular shape which is relatively long in the longitudinal direction P and formed in its middle in the transverse direction Q with a generally rectangular opening 171 largely cut out in the longitudinal direction P. Such film sheet 260 has an upper end section 266, as viewed in Fig. 6, adapted to be held by the clip 120, lateral sections 267a, 267b extending downward from the upper end section 266 on both sides of a central line $L_1$-$L_1$ bisecting a width dimension of the urine sensor 118, a lower end section 268a extending downward from the lower end of the lateral section 267a, and a lower end section 268b extending from the lower end section of the lateral section 267b. The lower end section 268a is connected with the lower end section 268b via a connecting region 268c. In the upper end section 266 of the film sheet 260, the electrodes 218a, 218b are exposed. In Fig. 6, a plurality of small circular regions in the lateral sections 267a, 267b and the lower end sections 268a, 268b indicate uncoated regions 169a, 169b formed in the insulating coating 170 in which a urine detecting region 175a or 175b of the electrode 218a or 218b are exposed.

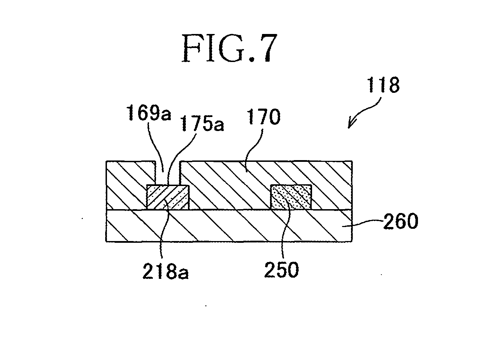

Fig. 7 is a sectional view taken along line VII-VII in Fig. 6. The film sheet 260 is formed on its upper surface with, in addition to the urine detecting electrode 218a, a resistive element 250 for detection of burnout and the urine detecting region 175a of the electrode 218a is exposed in the uncoated region 169a. The resistive element 250 is entirely covered with the insulating coating 170.

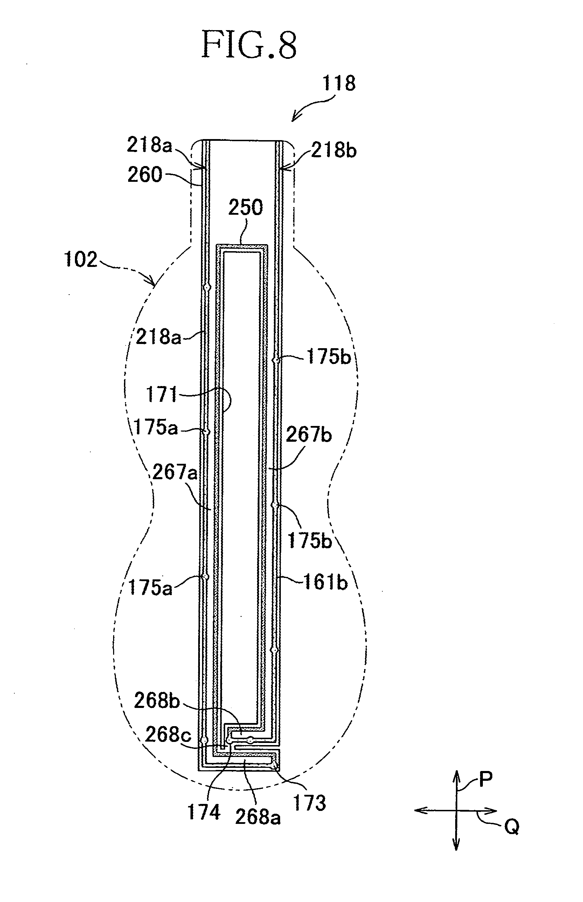

Fig. 8 is a plan view of the urine sensor 118 having the insulating coating peeled off. The film sheet 260 is formed on one surface with the generally L-shaped urine detecting electrode 218a extending along the lateral section 267a and the lower end section 268a and the inverted L-shaped urine detecting electrode 218b extending along the lateral section 267b and the lower end section 268b. The circular regions 175a, 175b of these electrodes 218a, 218b are exposed in the uncoated regions 169a, 169b shown in Fig. 6. The resistive element 250 for detection of burnout is formed between the electrodes 218a, 218b. The resistive element 250 is electrically connected to the respective lower end sections 173, 174 of the urine detecting electrodes 218a, 218b and extending along a peripheral edge of the opening 171.

As the film sheet 260 preferred to be used in such urine sensor 118, a polyester film having a thickness in a range of 50 to 100um may be used. The electrodes 218a, 218b may be obtained by printing the film sheet 260 with conductive ink or the other conductive coating material in a desired shape. The conductive ink or the conductive coating material may contain carbon black of 3 to 7% by mass, artificial graphite such as carbon graphite of 10 to 30% by mass and an appropriate quantity of silver powder. Each of the preferred electrodes 218a, 218b is designed to have a width dimension of 0.5 to 2mm and a resistance value of 150KΩ or less and has the uncoated regions 169a or 169b each having a diameter of 1 to 2mm. The burnout detecting resistance element 250 may be obtained by printing the film sheet 260 with ink, for example, containing carbon black of 3 to 7% by mass and artificial graphite of 5 to 10% by mass in a desired shape. This resistance element 250 has a resistance value substantially higher than that of the urine detecting electrodes 218a, 218b and the preferred resistance element 250 has a width dimension of 0.3 to 1mm and a resistance value in the order of 2 to 10MΩ. If the kind of ink used to form the resistance element 250 has conductive properties as high as the type of ink used form the electrodes 218a, 218b has and the resistance element 250 is dimensioned to have a sufficiently large width to make the process easy, a total length of the resistance element 250 may be dimensioned as long as possible so as to extend in the longitudinal direction P along the peripheral edge of the opening 171 and thereby to increase the resistance value thereof.

In the case of the urine receiver 102 using the urine sensor 118 shown in Fig. 6, the wiring 116 including the power source 116a normally carries faint current A from the source 116a via the clip 120 to the electrodes 218a, 218b and the resistance element 250 electrically connected to these electrodes 218a, 218b. As long as an electric circuit 110 including the wiring 116 carries electric current A, the urine detecting electrodes 218a, 218b is determined to be in a normal state. In contrast, if no current A is detected, it is determined that failures such as burnout occur in the urine detecting electrodes 218a, 218b and signals prompting exchange of the urine sensor 118 is output from the pump unit 108. Referring to Fig. 6, when a range enclosed by an imaginary line 176 is wetted with urine, the urine flows into the non-coated regions 169a, 169b and comes in contact with the urine detecting regions 175a, 175b to put the electrode 218a extending along the lateral section 267a and the electrode 218b extending along the lateral section 267b into the electrically connected state. When a resistance value between the electrodes 218a, 218b becomes lower than a resistance value of the resistance element 250, the electric current no more flows through the resistance element 250 but flows through urine of which the resistance value is relatively low. When a value of the electric current B flowing through urine becomes higher than the electric current A flowing through the resistance element 250, the electric circuit 110 detects such change of the current or an abrupt change of voltage or resistance value and thereby to detect occurrence of urination. In response to signals generated on the basis of such change, the pump is actuated to suck urine and the pump is stopped as the resistance value between the electrodes 218a, 218b increases again.

While the non-coated regions 169a, 169b serving to expose the urine detecting regions 175a, 175b are formed in the lateral sections 267a, 267b asymmetrically about the center line $L_1$-$L_1$ in the urine sensor 118 shown in Fig. 6, it is also possible to form these non-coated regions 169a, 169b symmetrically about the center line $L_1$-$L_1$. In the lower end sections 268a, 268b of the film sheet 260 and in the vicinity of them, a plurality of the non-coated regions 169a, 169b narrowly spaced one from another. With the non-coated regions 169a, 169b arranged in this manner, even if urine immediately after discharged quickly spreads downward from above as viewed in Fig. 6 before reaching the air permeable-resistant sheet 124, the urine receiver 102 is capable of quickly sucking such urine. Alternatively, the non-coated regions 169a, 169b may be provided at appropriate position in the lateral sections 267a, 267b depending on the posture supposed to be taken by the wearer when the urine receiver 102 is put on the wearer's body.

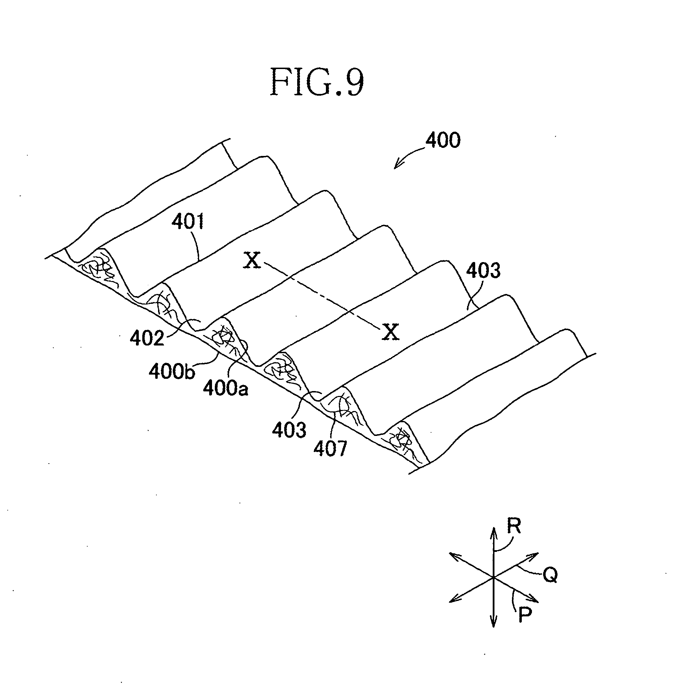

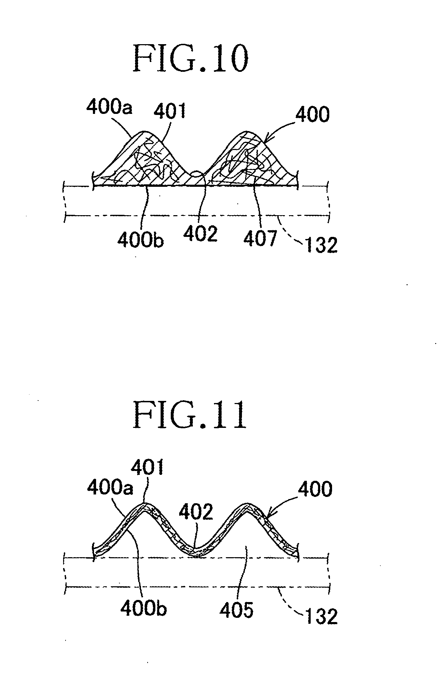

Fig. 9 is a perspective view of a sheet 400 as an example of the skin-contact sheet 134 and Fig. 10 is a sectional view taken along line X-X in Fig. 9. The sheet 400 is formed of a nonwoven fabric containing hydrophobic thermoplastic synthetic fibers of 80 to 100% by mass and liquid-absorbent fibers such as rayon fibers of 20 to 0% by mass wherein these fibers are fusion-bonded or interlaced together. The sheet 400 has an outer surface 400a facing the wearer's skin and an inner surface 400b facing the urine sensor 108 wherein a longitudinal direction, a transverse direction and a thickness direction correspond to the longitudinal direction P, the transverse direction Q and the thickness direction R of the urine receiver 102, respectively. The outer surface 400a and the inner surface 400b correspond to the upper surface and the lower surface, respectively, in Fig. 4.

The outer surface 400a of the sheet 400 has undulation formed of crests 401 and troughs 402 alternating in the longitudinal direction P and the crests 401 and the troughs 402 extending in the transverse direction Q in parallel one to another. The inner surface 40b of the sheet 400 is flat and put in close contact with the filter 132. The sheet 400 is used in a manner that the crests 401 and the troughs 402 may extend across a pair of the electrodes 218a, 218b in the urine sensor 118 and side edges 403 opposed in the transverse direction Q may be preferably located inside the respective leakage-barriers 136 (See Fig. 4) provided on both sides of the urine receiver 102.

With the urine receiver 102 using the sheet 400 as the skin-contact sheet 134 and put on the wearer's body, the urine discharged toward the sheet 400 is apt to be collected in the troughs 402 and then to spread in the transverse direction Q. In the urine receiver 102, therefore, the paired electrodes 218a, 218b can be quickly connected via urine and thereupon the urine receiver 102 can start to suck the discharged urine. The urine receiver 102 utilizing function of the troughs 402 of the sheet 400 is advantageous particularly in that, even when the wearer is in the recumbent position, the discharged urine spreads in the transverse direction Q and the paired electrodes 218a, 218b can be quickly connected via urine.

The urine detecting regions 175a, 175b (See Fig. 6) in the electrodes 218a, 218b used in combination with the sheet 400 functioning in the manner as described above are preferably formed symmetrically about the center line $L_1$-$L_1$. The sheet 400 has a basis mass in a range of 15 to 80g/m$^2$ and comprises a plurality of hydrophobic thermoplastic synthetic fibers 407 each having a fineness in a range of 1 to 8dtex and a fiber length in a range of 20 to 80mm wherein most of the thermoplastic synthetic fibers 407 are preferably oriented in the transverse direction Q. Such sheet 400 can be obtained by heating and pressing a nonwoven fabric such as a thermally bonded nonwoven fabric between vertically paired molds wherein the upper mold has the crests and the troughs and the lower mold is flat. A machine direction in the course of producing the nonwoven fabric may be aligned with the direction in which the troughs extend to orient the thermoplastic synthetic fibers in the transverse direction Q. The sheet 400 may be previously treated to become more hydrophilic in the troughs 402 than in the crests 401 to promote the movement of the discharged urine from the crests 401 to the troughs 402. If desired, it is also possible to make the sheet 400 so that respective tops of the crests 401 are water-repellent and respective bottoms of the troughs 402 are hydrophilic.

Fig. 11 is a view similar to Fig. 10, exemplarily showing the sheet 400 which is different from those shown in Figs. 9 and 10. The sheet 400 shown in Fig. 11 has a generally uniform thickness over its entire area, i.e., the inner surface 400b extends in parallel to the outer surface 400a, and has crests and troughs 411, 412 corresponding to the those 401, 402 of the sheet shown in Figs. 9 and 10. The inner surface 400b are intermittently bonded or heat-sealed along respective bottoms of the troughs 412 to the filter 132 facing the inner surface 400b of the sheet 400. In this manner, the sheet 400 is joined to the filter 132 intermittently in the transverse direction Q and the sheet 400 and the filter 132 are put in close contact with each other in a region defined between each pair of the adjacent joints so as to facilitate passage of urine through the sheet 400 and the filter 132. Tunnels 405 extending in the transverse direction Q are defined between the sheet 400 and the filter 132 and therefore the urine receiver 102 having a feature shown in Fig. 11 assures an air-permeability higher than the urine receiver 102 having a feature shown in Figs. 9 and 10. The sheet 400 exemplarily shown in Fig. 11 may be obtained by sandwiching a nonwoven fabric such as a thermally bonded nonwoven fabric between each pair of heated molds from above and below. These paired molds may have the crests and troughs, respectively, adapted to be engaged one with another.

The sheet exemplarily shown in Figs. 10 and 11 is used not only as the skin-contact sheet 134 but also used so as to lie between the skin-contact sheet 134 and filter 132 in Fig. 4. When the fiber interstice in the sheet 400 is comparable to the fiber interstice in the filter 132, the filter 132 may be replaced by the sheet 400. In any case, the sheet 400 lies outside the electrodes 218a, 218b in the depth direction R and the crests 401 and the troughs 402 extend across these electrodes 218a, 218b. With such an arrangement, the urine receiver 102 allows these electrodes 218a, 218b to be electrically connected with each other as quickly as possible. It should be appreciated that the dispersant sheet 126 may be eliminated if the air permeable-resistant sheet 124 contains, in addition to the thermoplastic synthetic fibers, hydrophilic fiber such as rayon fiber and thereby promotes dispersion of urine.

## Claims

1. A urine receiver (102) comprising a leakage-barrier receptacle (112) having a bottom (112b) and a peripheral wall (112c) and a top opening (112a), a urine evacuation duct extending through said peripheral wall (112c) and adapted to be connected with urine suction means (100a) provided separately of said receptacle (112), a plurality of liquid-pervious sheets (134, 126) stacked one on another to cover said top opening (112a) and a pair of electrodes (143) spaced from each other and extending in one direction in parallel to each other wherein, in response to electrical connection of said pair of electrodes (143) via urine contained in one or more of said plurality of liquid-pervious sheets (134, 126), said urine suction means (100a) is actuated to suck the urine discharged by the wearer into said receptacle (112) through said top opening (112a) and to evacuate said urine sucked into said receptacle (112) from said receptacle (112) through said urine evacuation duct,

wherein any one of said plurality of liquid-pervious sheets (134, 126) lying outside said pair of electrodes (143) in a depth direction of said receptacle (112) is formed of a nonwoven fabric containing thermoplastic synthetic fibers and has an outer surface (400a) facing outward in said depth direction and an inner surface (400b) facing inward in said depth direction said urine receiver (102) being **characterized in that**: said outer surface (400a) is formed with undulations comprising crests (401) and troughs (402) alternating in said one direction and each of said crests (401) and troughs (402) extends in a direction intersecting with said one direction across said pair of electrodes (143).

2. The urine receiver according to claim 1, wherein said sheets formed on the outer surface thereof with said undulation has a flat inner surface and is put in close contact with said liquid-pervious sheet facing said flat inner surface.

3. The urine receiver according to claim 1, wherein said sheet formed on said outer surface thereof with said undulation has said inner surface extending in parallel to said outer surface and is put in close contact with said liquid-pervious sheet facing said inner surface along respective bottoms of said troughs.

4. The urine receiver according to claims 1 through 3, wherein, respective tops of said crests are hydrophobic and respective bottoms of said troughs are hydrophilic.

5. The urine receiver according to claims 1 through 4, wherein said nonwoven fabric contains in said troughs thermoplastic synthetic fibers extending in said direction orthogonal to said one direction.

**Patentansprüche**

1. Urinfänger (102), umfassend eine Austrittschutzaufnahme (112) mit einem Boden (112b), einer Umfangswand (112c) und einer oberen Öffnung (112a), weiter umfassend einen Urinentleerungskanal, der sich durch die besagte Umfangswand (112c) erstreckt und zum Anschluss an Urinsaugmittel (100a) ausgelegt ist, die separat von der besagten Aufnahme (112) vorgesehen sind, weiter umfassend eine Vielzahl von flüssigkeitsdurchlässigen Lagen (134, 126), die zum Abdecken der besagten oberen Öffnung (112a) aufeinander gestapelt sind, und weiter umfassend ein Paar von voneinander beabstandeten Elektroden (143), die sich parallel zueinander in einer Richtung erstrecken, wobei, als Reaktion auf die elektrische Verbindung des besagten Paares von Elektroden (143) über in einer oder mehreren der besagten Vielzahl von flüssigkeitsdurchlässigen Lagen (134, 126) enthaltenen Urin, das besagte Urinsaugmittel (100a) betätigt wird, um den vom Träger abgegebenen Urin durch die besagte obere Öffnung (112a) in die besagte Aufnahme (112) zu saugen und den in die besagte Aufnahme (112) gesaugten Urin aus der besagten Aufnahme (112) durch den besagten Urinentleerungskanal zu entleeren,
wobei eine beliebige der besagten Vielzahl von flüssigkeitsdurchlässigen Lagen (134, 126), die in einer Tiefenrichtung der besagten Aufnahme (112) außerhalb des besagten Paares von Elektroden (143) liegen, aus einem Faservlies gebildet ist, das thermoplastische Kunstfasern enthält und eine in der besagten Tiefenrichtung nach außen gekehrte Außenfläche (400a) und eine in der besagten Tiefenrichtung nach innen gekehrte Innenfläche (400b) aufweist, wobei der besagte Urinfänger (102) **dadurch gekennzeichnet ist, dass** die besagte Außenfläche (400a) eine Welligkeit mit Erhöhungen (401) und Vertiefungen (402) aufweist, die sich in der besagten einen Richtung abwechseln, und wobei die besagten Erhöhungen (401) und Vertiefungen (402) sich jeweils in einer Richtung erstrecken, die sich mit der besagten einen Richtung über das besagte Paar von Elektroden (143) schneiden.

2. Urinfänger nach Anspruch 1, wobei die besagte auf der Außenfläche gebildete Lage mit der besagten Welligkeit eine flache Innenfläche aufweist und in engem Kontakt mit der besagten der besagten flachen Innenfläche zugekehrten flüssigkeitsdurchlässigen Lage ist.

3. Urinfänger nach Anspruch 1, wobei die besagte auf der Außenfläche gebildete Lage mit der besagten Welligkeit die besagte Innenfläche aufweist, die sich parallel zur besagten Außenfläche erstreckt und entlang den betreffenden Böden der besagten Vertiefungen in engem Kontakt mit der besagten der besagten flachen Innenfläche zugekehrten flüssigkeitsdurchlässigen Lage ist.

4. Urinfänger nach einem der Ansprüche 1 bis 3, wobei die betreffenden Oberseiten der besagten Erhöhungen wasserabweisend und die betreffenden Böden der besagten Vertiefungen wasseranziehend sind.

5. Urinfänger nach einem der Ansprüche 1 bis 4, wobei das besagte Faservlies in den besagten Vertiefungen thermoplastische Kunstfasern enthält, die in der besagten senkrecht zur besagten einen Richtung stehenden Richtung verlaufen.

## Revendications

1. Collecteur d'urine (102) qui comporte un récipient de protection antifuite (112), ayant un fond (112b) et une paroi périphérique (112e) et une ouverture supérieure (112a), une conduite d'évacuation d'urine s'étendant au travers de ladite paroi périphérique (112c) et étant adaptée à des fins de raccordement avec le moyen d'aspiration d'urine (100a) mis en oeuvre séparément par rapport audit récipient (112), une pluralité de feuilles perméables aux liquides (134, 126) empilées les unes sur les autres afin de recouvrir ladite ouverture supérieure (112a) et une paire d'électrodes (143) espacées l'une par rapport à l'autre et s'étendant dans une direction de manière parallèle l'une par rapport à l'autre dans lequel, en réponse à une connexion électrique de ladite paire d'électrodes (143) par le biais de l'urine contenue dans une ou plusieurs de ladite pluralité de feuilles perméables aux liquides (134, 126), ledit moyen d'aspiration d'urine (100a) est actionné à des fins d'aspiration de l'urine déchargée par l'utilisateur à l'intérieur dudit récipient (112) au travers de ladite ouverture supérieure (112a) et à des fins d'évacuation de ladite urine aspirée dans ledit récipient (112) depuis ledit récipient (112) au travers de ladite conduite d'évacuation d'urine,
dans lequel l'une quelconque de ladite pluralité de feuilles perméables aux liquides (134, 126) reposant à l'extérieur de ladite paire d'électrodes (143) dans une direction allant dans le sens de la profondeur dudit récipient (112) est formée à partir d'un tissu non tissé contenant des fibres synthétiques thermoplastiques et a une surface extérieure (400a) orientée vers l'extérieur dans ladite direction allant dans le sens de la profondeur et une surface intérieure (400b) orientée vers l'intérieur dans ladite direction allant dans le sens de la profondeur, ledit collecteur d'urine (102) étant **caractérisé en ce que** : ladite surface extérieure (400a) est formée avec des ondulations comportant des crêtes (401) et des creux (402) alternant dans ladite une direction et chacun parmi lesdites crêtes (401) et lesdits creux (402) s'étend dans une direction croisant ladite une direction en travers de ladite paire d'électrodes (143).

2. Collecteur d'urine selon la revendication 1, dans lequel ladite feuille formée sur la surface extérieure de celui-ci avec ladite ondulation a une surface intérieure plate et est mise en contact étroit avec ladite feuille perméable aux liquides orientée vers ladite surface intérieure plate.

3. Collecteur d'urine selon la revendication 1, dans lequel ladite feuille formée sur ladite surface extérieure de celui-ci avec ladite ondulation a ladite surface intérieure s'étendant de manière parallèle par rapport à ladite surface extérieure et est mise en contact étroit avec ladite feuille perméable aux liquides orientée vers ladite surface intérieure le long des fonds respectifs desdits creux.

4. Collecteur d'urine selon l'une quelconque des revendications 1 à 3, dans lequel des parties supérieures respectives desdites crêtes sont hydrophobes et des fonds respectifs desdits creux sont hydrophiles.

5. Collecteur d'urine selon l'une quelconque des revendications 1 à 4, dans lequel ledit tissu non tissé contient dans lesdits creux des fibres synthétiques thermoplastiques s'étendant dans ladite direction perpendiculaire à ladite une direction.

FIG.1
108
pump unit
116
116a
106a
100a
106
104
110
120
118
102b
102a
114
112c
112
102
100

FIG.2

116
300
120
106
118
302
304
303
102
301

FIG.3
V
144
143a
143b
142b
218a
218b
118
102
138
134
136
IV
IV
P
136
Q
140
145b
142a
144
V

102
136b
118
136b
134a
136c
136
136d
218a
218b
136d
136
136c
260
134
130
132
102b
128
171
124
134b
126
R
124a
122
112d
122
102a
142a
152
152
112c
142b
143a
112a
112
143b
112b
142d
142c
144
Q

# FIG.5

122
260
138
118
126
P
R
152
112
114
112a
144
134b
134a
112b
112c
118
112d
152
140
134
130
132
128
124
122
142b
142a
145b

# FIG.6

L1
266
218a
218b
118
260
169a
170
175a
102
169b
171
267a
267b
176
169a
175b
VII
VII
175a
169b
175b
169a
169b
268b
268c
169a
175a
P
268a
Q
L1

FIG.7

118
169a
175a
170
260
218a
250

FIG.8

118
218a
218b
260
250
102
218a
175b
171
175a
267b
267a
175b
175a
161b
268b
268c
174
268a
173
P
Q

FIG.9

400
401
X
403
X
402
400b
400a
403
407

R
Q
P

FIG.10

400a
401
400
400b
402
407
132

FIG.11

401
400a
400
402
400b
405
132

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2007038194 A1
- JP 2006026108 A